# FASCICULE DE BREVET EUROPEEN

(11) **EP 3 207 011 B1**
(45) Date de publication et mention de la délivrance du brevet: **19.05.2021**
(21) Numéro de dépôt: 15798490.7
(22) Date de dépôt: 07.10.2015
(51) Int. Cl.: C07C 17/20, C07C 17/25, C07C 19/01, C07C 21/18

(54) **COMPOSITIONS A BASE DE 1,1,1,3,3-PENTACHLOROPROPANE**
ZUSAMMENSETZUNGEN MIT 1,1,1,3,3-PENTACHLOROPROPAN
COMPOSITIONS CONTAINING 1,1,1,3,3-PENTACHLOROPROPANE

(30) Priorité: 16.10.2014 FR 1459928
(43) Date de publication de la demande: 23.08.2017
(73) Titulaire: Arkema France, 92700 Colombes (FR)
(72) Inventeur: PIGAMO, Anne, 69340 Francheville (FR); DEUR-BERT, Dominique, 69390 Charly (FR); WENDLINGER, Laurent, 69510 Soucieu en Jarrest (FR); COLLIER, Bertrand, 69230 Saint-Genis-Laval (FR)
(74) Mandataire: Arkema Patent
(86) Numéro de dépôt international: PCT/FR2015/052694
(87) Numéro de publication internationale: WO 2016/059323

(56) Documents cités:
- EP-A1- 2 341 040
- WO-A1-2012/098421
- WO-A1-2014/094590
- WO-A2-2012/094288
- US-A- 5 705 779
- US-A1- 2012 059 199
- US-A1- 2012 184 786

## Description

### DOMAINE DE L'INVENTION

La présente invention concerne des compositions à base de F-240fa (1,1,1,3,3-pentachloropropane) ainsi que leur utilisation notamment pour la production de F-1233zdE (trans-1-chloro-3,3,3-trifluoropropène) et/ou de F-1234zeE (trans-1,3,3,3-tétrafluoropropène).

### ARRIERE-PLAN TECHNIQUE

Les fluorooléfines F-1233zdE et F-1234zeE sont des composés d'intérêt majeur pour les systèmes de réfrigération et de climatisation, compte tenu des nouvelles réglementations environnementales.

Il est connu de produire les hydrofluorooléfines telles que le F-1233zdE et/ou le F-1234zeE par fluoration d'hydrochlorooléfines ou d'hydrochlorocarbures notamment. Cette fluoration est généralement une fluoration catalytique utilisant l'acide fluorhydrique comme agent fluorant.

Parmi les voies permettant d'obtenir le F-1233zdE, il est en particulier connu d'utiliser le F-240fa (1,1,1,3,3-pentachloropropane) en tant que composé de départ. Il est fait référence par exemple au document US 8,704,017 à cet égard qui, décrit un procédé de fluoration en phase liquide en l'absence de catalyseur.

Un autre procédé possible est la fluoration en phase gazeuse en présence d'un catalyseur et d'un agent oxydant tel que le chlore par exemple, pour maintenir la stabilité du catalyseur.

Par ailleurs, il est connu d'utiliser le composé F-1233zdE pour l'obtention successive du F-1234zeE. Il est fait référence par exemple au document US 5,895,825 à cet égard.

On connait par EP 2 341 040 un procédé de production de 1,3,3,3-tétrafluoropropène par réaction du 1,1,1,3,3-pentachloropropane avec HF. WO 2014/094590 décrit également un procédé de coproduction de HFO-1234ze et HFC-245fa par fluoration en phase gazeuse en utilisant HCC-240fa comme matière première. US 2012/0184786 décrit un procédé intégré de coproduction de trans-1-chloro-3,3,3-trifluoropropène, trans-1,3,3,3-tétrafluoropropène et 1,1,1,3,3-pentafluoropropane. WO 2012/098421 décrit un procédé de fluoration comprenant une étape d'activation et une étape de réaction pour produire un composé fluoré. US 2012/0059199 décrit un procédé de production du HCFO-1233zdE en continu en l'absence de catalyseur. US 5,705,779 décrit un procédé de préparation du HCC-240fa par photochloration du HCC-250. WO 2012/094288 décrit un procédé pour produire du HCFO-1233zdE.

Il est souhaitable de parvenir à produire du F-1233zdE présentant une teneur faible en impuretés. En particulier, la formation de certaines impuretés toxiques et/ou inflammables et/ou susceptible de polymériser et/ou qui sont difficiles à séparer du F-1233zdE doit être minimisée.

Il est également souhaitable de parvenir à produire du F-1234zeE présentant une teneur faible en impuretés. En particulier, la formation de certaines impuretés toxiques et/ou susceptible de polymériser et/ou qui sont difficiles à séparer du F-1234zeE doit être minimisée.

Il existe donc un besoin de fournir des moyens permettant d'obtenir des compositions de F-1233zdE et de F-1234zeE de pureté satisfaisante.

### RESUME DE L'INVENTION

L'invention concerne en premier lieu une composition comprenant au moins 99 % en poids de 1,1,1,3,3-pentachloropropane, et comprenant au moins un composé choisi parmi une liste de composés supplémentaires consistant en les dichloropropanes, les trichloropropanes, les tétrachloropropanes, les hexachloropropanes, les heptachloropropanes, les chloropropènes, les dichloropropènes, les trichloropropènes, les tétrachloropropènes, les pentachloropropènes et l'hexachloropropène, ledit composé étant présent dans la composition en une teneur pondérale inférieure ou égale à 500 ppm.

Selon un mode de réalisation, ledit composé est présent dans la composition en une teneur pondérale inférieure ou égale à 250 ppm ; de préférence inférieure ou égale à 150 ppm ; plus particulièrement inférieure ou égale à 100 ppm ; plus particulièrement inférieure ou égale à 50 ppm ; et idéalement inférieure ou égale à 10 ppm

Selon un mode de réalisation, la composition comprend une pluralité de composés choisis parmi ladite liste de composés supplémentaires, chacun des composés de ladite pluralité de composés étant présent dans la composition en une teneur pondérale inférieure ou égale à 500 ppm ; de préférence inférieure ou égale à 250 ppm ; de préférence inférieure ou égale à 150 ppm ; plus particulièrement inférieure ou égale à 100 ppm ; plus particulièrement inférieure ou égale à 50 ppm ; et idéalement inférieure ou égale à 10 ppm.

Selon un mode de réalisation, la composition comprend une pluralité de composés choisis parmi ladite liste de composés supplémentaires, la teneur pondérale totale de l'ensemble des composés de ladite liste étant inférieure ou égale à 1000 ppm ; de préférence inférieure ou égale à 500 ppm ; de préférence inférieure ou égale à 250 ppm ; de préférence inférieure ou égale à 150 ppm ; plus particulièrement inférieure ou égale à 100 ppm ; plus particulièrement inférieure ou égale à 50 ppm ; et idéalement inférieure ou égale à 10 ppm.

Selon un mode de réalisation, la composition comprend au moins 99,5 % en poids, et de préférence au moins 99,8 % en poids, et de manière plus particulièrement préférée au moins 99,9 % en poids, de 1,1,1,3,3-pentachloropropane.

Selon un mode de réalisation, la composition comprend au moins un composé choisi parmi le groupe constitué de l'hexachloropropène et des heptachloropropanes, et la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

Selon un mode de réalisation, la composition comprend au moins un composé choisi parmi le groupe constitué des pentachloropropènes et des hexachloropropanes, et la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

Selon un mode de réalisation, la composition comprend au moins un composé choisi parmi le groupe constitué des tétrachloropropènes, et la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

Selon un mode de réalisation, la composition comprend au moins un composé choisi parmi le groupe constitué du 2,3,3,3-tétrachloropropène, du 1,1,2,3-tétrachloropropène, et la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

Selon un mode de réalisation, la composition comprenant au moins un composé choisi parmi le groupe constitué des trichloropropènes et des tétrachloropropanes, et la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

Selon un mode de réalisation, la composition comprend au moins un composé choisi parmi le groupe constitué du 1,1,3-trichloropropène, du 3,3,3-trichloropropène, du 1,1,1,3-tétrachloropropane, du 1,1,2,3-tétrachloropropane et du 1,1,1,2-tétrachloropropane, et la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

L'invention concerne également un procédé de production de 1,3,3,3-tétrafluoropropène, notamment sous forme trans, comprenant :
- la fourniture d'une composition telle que définie ci-dessus ;
- la réaction de cette composition avec de l'acide fluorhydrique, de préférence en phase gazeuse.

Selon un mode de réalisation, le procédé comprend une unique étape de fluoration catalytique.

Selon un mode de réalisation, le procédé comprend deux étapes successives de fluoration catalytique, à savoir :
- la réaction de la composition avec de l'acide fluorhydrique en phase gazeuse, pour fabriquer un produit intermédiaire ;
- optionnellement, une purification du produit intermédiaire ; puis
- la réaction du produit intermédiaire avec de l'acide fluorhydrique en phase gazeuse, pour fabriquer le 1,3,3,3-tétrafluoropropène ;
le produit intermédiaire étant de préférence le 1-chloro-3,3,3-trifluoropropène, notamment sous forme trans.

La présente invention permet de surmonter les inconvénients de l'état de la technique. Elle fournit plus particulièrement des compositions à base de F-240fa dont la teneur en impuretés permet de minimiser la présence d'impuretés néfastes dans le F-1233zdE ou dans le F-1234zeE fabriqués à partir de celles-ci.

En effet, les impuretés présentes dans le F-1233zdE ou dans le F-1234zeE dépendent pour partie des impuretés initialement présentes dans le F-240fa qui est utilisé pour les fabriquer. Au cours de la ou des réactions de fluoration, certaines des impuretés du F-240fa peuvent être converties en impuretés différentes dans le F-1233zdE ou dans le F-1234zeE. La maitrise des impuretés présentes dans le F-240fa permet donc indirectement de contrôler les impuretés présentes dans le F-1233zdE et dans le F-1234zeE.

Un tel contrôle indirect peut être avantageux dans la mesure où les impuretés du F-1233zdE peuvent être plus difficiles à séparer du F-1233zdE que les impuretés du F-240fa par rapport au F-240fa ; et où les impuretés du F-1234zeE peuvent être plus difficiles à séparer du F-1234zeE que les impuretés du F-240fa par rapport au F-240fa. C'est notamment le cas lorsque les impuretés du F-1233zdE (respectivement les impuretés du F-1234zeE) ont un point d'ébullition très proche ou forment un azéotrope ou un quasi-azéotrope avec F-1233zdE (respectivement le F-1234zeE).

### DESCRIPTION DE MODES DE REALISATION DE L'INVENTION

L'invention est maintenant décrite plus en détail et de façon non limitative dans la description qui suit.

Toutes les teneurs indiquées sont des teneurs pondérales sauf mention contraire.

### Nomenclature

Le tableau suivant fournit la nomenclature d'un certain nombre de composés qui interviennent dans l'invention.

| **Formule** | **Notation** | **Nom complet** |
|---|---|---|
| CCl₃-CHCl-CCl₃ | F-220da | 1,1,1,2,3,3,3-heptachloropropane |
| CHCl₂-CCl₂-CCl₃ | F-220aa | 1,1,1,2,2,3,3-heptachloropropane |
| CF₃-CHCl-CF₃ | F-226da | 2-chloro-1,1,1,3,3,3-hexafluoropropane |
| CF₃-CHF-CClF₂ | F-226ea | 1-chloro-1,1,2,3,3,3-hexafluoropropane |
| CF₃-CFCl-CHF₂ | F-226ba | 2-chloro-1,1,2,3,3,3-hexafluoropropane |
| CF₃-CF₂-CHFCl | F-226ca | 3-chloro-1,1,1,2,2,3-hexafluoropropane |
| CClF₂-CF₂-CHF₂ | F-226cb | 1-chloro-1,1,2,2,3,3-hexafluoropropane |
| CCl₃-CH₂-CCl₃ | F-230fa | 1,1,1,3,3,3-hexachloropropane |
| CHCl₂-CHCl-CCl₃ | F-230da | 1,1,1,2,3,3-hexachloropropane |
| CHCl₂-CCl₂-CHCl₂ | F-230aa | 1,1,2,2,3,3-hexachloropropane |
| CH₂Cl-CCl₂-CCl₃ | F-230ab | 1,1,1,2,2,3-hexachloropropane |
| CF₃-CH₂-CF₃Cl | F-235fa | 3-chloro-1,1,1,3,3-pentafluoropropane |
| CF₃-CHF-CHFCl | F-235ea | 1-chloro-1,2,3,3,3-pentafluoropropane |
| CHF₂-CHF-CClF₂ | F-235eb | 1-chloro-1,1,2,3,3-pentafluoropropane |
| CHClF-CF₂-CHF₂ | F-235ca | 3-chloro-1,1,2,2,3-pentafluoropropane |
| CH₂Cl-CF₂-CF₃ | F-235cb | 3-chloro-1,1,1,2,2-pentafluoropropane |
| CH₂F-CF₂-CClF₂ | F-235cc | 1-chloro-1,1,2,2,3-pentafluoropropane |
| CHF₂-CHCl-CF₃ | F-235da | 2-chloro-1,1,1,3,3-pentafluoropropane |
| CHF₂-CClF-CHF₂ | F-235ba | 2-chloro-1,1,2,3,3-pentafluoropropane |
| CH₂F-CClF-CF₃ | F-235bb | 2-chloro-1,1,1,2,3-pentafluoropropane |
| CF₃-CH₂-CF₃ | F-236fa | 1,1,1,3,3,3-hexafluoropropane |
| CHF₂-CF₂-CHF₂ | F-236ca | 1,1,2,2,3,3-hexafluoropropane |
| CH₂F-CF₂-CF₃ | F-236cb | 1,1,1,2,2,3-hexafluoropropane |
| CHF₂-CHF-CF₃ | F-236ea | 1,1,1,2,3,3-hexafluoropropane |
| CHCl₂-CH₂-CCl₃ | F-240fa | 1,1,1,3,3-pentachloropropane |
| CHCl₂-CHCl-CHCl₂ | F-240da | 1,1,2,3,3-pentachloropropane |
| CH₂Cl-CHCl-CCl₃ | F-240db | 1,1,1,2,3-pentachloropropane |
| CH₂Cl-CCl₂-CHCl₂ | F-240aa | 1,1,2,2,3-pentachloropropane |
| CH₃-CCl₂-CCl₃ | F-240ab | 1,1,1,2,2-pentachloropropane |
| CH₂F-CF₂-CHF₂ | F-245ca | 1,1,2,2,3-pentafluoropropane |
| CF₃-CF₂-CH₃ | F-245cb | 1,1,1,2,2-pentafluoropropane |
| CHF₂-CHF-CHF₂ | F-245ea | 1,1,2,3,3-pentafluoropropane |
| CH₂F-CHF-CF₃ | F-245eb | 1,1,1,2,3-pentafluoropropane |
| CHF₂-CH₂-CF₃ | F-245fa | 1,1,1,3,3-pentafluoropropane |
| CHCl₂-CH₂-CHCl₂ | F-250fa | 1,1,3,3-tétrachloropropane |
| CH₂Cl-CH₂-CCl₃ | F-250fb | 1,1,1,3-tétrachloropropane |
| CH₂Cl-CHCl-CHCl₂ | F-250da | 1,1,2,3-tétrachloropropane |
| CH₃-CHCl-CCl₃ | F-250db | 1,1,1,2-tétrachloropropane |
| CH₂Cl-CCl₂-CH₂Cl | F-250aa | 1,2,2,3-tétrachloropropane |
| CH₃-CCl₂-CHCl₂ | F-250ab | 1,1,2,2-tétrachloropropane |
| CF₂Cl-CH₂-CH₂F | F-253fa | 1-chloro-1,1,3-trifluoropropane |
| CH₂Cl-CH₂-CF₃ | F-253fb | 1-chloro-3,3,3-trifluoropropane |
| CF₂Cl-CH₂-CH₂F | F-253fc | 1-chloro-1,1,3-trifluoropropane |
| CH₂F-CClF-CH₂F | F-253ba | 2-chloro-1,2,3-trifluoropropane |
| CHF₂-CClF-CH₃ | F-253bb | 2-chloro-1,1,2-trifluoropropane |
| CH₂Cl-CF₂-CH₂F | F-253ca | 1-chloro-2,2,3-trifluoropropane |
| CHFCl-CF₂-CH₃ | F-253cb | 1-chloro-1,2,2-trifluoropropane |
| CHF₂-CHF-CH₂Cl | F-253ea | 3-chloro-1,1,2-trifluoropropane |
| CHClF-CHF-CH₂F | F-253eb | 1-chloro-1,2,3-trifluoropropane |
| CClF₂-CHF-CH₃ | F-253ec | 1-chloro-1,1,2-trifluoropropane |
| CH₂Cl-CH₂-CHCl₂ | F-260fa | 1,1,3-trichloropropane |
| CH₃-CH₂-CCl₃ | F-260fb | 1,1,1-trichloropropane |
| CH₂Cl-CHCl-CH₂Cl | F-260da | 1 ,2, 3-trich loropropane |
| CH₃-CHCl-CHCl₂ | F-260db | 1,1,2-trichloropropane |
| CH₃-CCl₂-CH₂Cl | F-260aa | 1,2,2-trichloropropane |
| CH₂Cl-CH₂-CH₂Cl | F-270fa | 1,3-dichloropropane |
| CH₃-CH₂-CHCl₂ | F-270fb | 1,1-dichloropropane |
| CH₃-CHCl-CH₂Cl | F-270da | 1,2-dichloropropane |
| CH₃-CCl₂-CH₃ | F-270aa | 2,2-dichloropropane |
| CCl₃-CCl=CCl₂ | F-1210xa | hexachloropropène |
| CF₃-CCl=CCl₂ | F-1213xa | 1,1,2-trichloro-3,3,3-trifluoropropène |
| CF₂Cl-CCl=CFCl | F-1213xb | 1,2,3-trichloro-1,3,3-trifluoropropène |
| CFCl₂-CCl=CF₂ | F-1213xc | 2,3,3-trichloro-1,1,3-trifluoropropène |
| CCl₃-CF=CF₂ | F-1213yc | 3,3,3-trichloro-1,1,2-trifluoropropène |
| CFCl₂-CF=CFCl | F-1213yb | 1,3,3-trichloro-1,2,3-trifluoropropène |
| CF₂Cl-CF=CCl₂ | F-1213ya | 1,1,3-trichloro-2,3,3-trifluoropropène |
| CCl₂F-CF=CF₂ | F-1214yc | 3,3-dichloro-1,1,2,3-tétrafluoropropène |
| CClF₂-CCl=CF₂ | F-1214xc | 2,3-dichloro-1,1,3,3-tétrafluoropropène |
| CClF₂-CF=CFCl | F-1214yb | 1,3-dichloro-1,2,3,3-tétrafluoropropène |
| CF₃-CCl=CFCl | F-1214xb | 1,2-dichloro-1,3,3,3-tétrafluoropropène |
| CF₃-CF=CCl₂ | F-1214ya | 1,2-dichloro-2,3,3,3-tétrafluoropropène |
| CF₃-CCl=CF₂ | F-1215xc | 2-chloro-1,1,3,3,3-pentafluoropropène |
| CF₂Cl-CF=CF₂ | F-1215yc | 3-chloro-1,1,2,3,3-pentafluoropropène |
| CF₃-CF=CFCl | F-1215yb | 1-chloro-1,2,3,3,3-pentafluoropropène |
| CF₃-CF=CF₂ | F-1216yc | hexafluoropropène |
| CHCl₂-CCl=CCl₂ | F-1220xa | 1,1,2,3,3-pentachloropropène |
| CCl₃-CCl=CHCl | F-1220xd | 1,2,3,3,3-pentachloropropène |
| CCl₃-CH=CCl₂ | F-1220za | 1,1,3,3,3-pentachloropropène |
| CF₃-CCl=CHCl | F-1223xd | 1,2-dichloro-3,3,3-trifluoropropène |
| CF₂Cl-CCl=CHF | F-1223xe | 2,3-dichloro-1,3,3-trifluoropropène |
| CHFCl-CCl=CF₂ | F-1223xc | 2,3-dichloro-1,1,3-trifluoropropène |
| CFCl₂-CH=CF₂ | F-1223zc | 3,3-dichloro-1,1,3-trifluoropropène |
| CF₂Cl-CH=CFCl | F-1223zb | 1,3-dichloro-1,3,3-trifluoropropène |
| CF₃-CH=CCl₂ | F-1223za | 1,1-dichloro-3,3,3-trifluoropropène |
| CHF₂-CF=CCl₂ | F-1223ya | 1,1-dichloro-2,3,3-trifluoropropène |
| CF₂Cl-CF=CHCl | F-1223yd | 1,3-dichloro-2,3,3-trifluoropropène |
| CFCl₂-CF=CHF | F-1223ye | 3,3-dichloro-1,2,3-trifluoropropène |
| CHCl₂-CF=CF₂ | F-1223yc | 3,3-dichloro-1,1,2-trifluoropropène |
| CHFCl-CF=CF₂ | F-1224yc | 3-chloro-1,1,2,3-tétrafluoropropène |
| CHF₂-CCl=CF₂ | F-1224xc | 2-chloro-1,1,3,3-tétrafluoropropène |
| CF₂Cl-CH=CF₂ | F-1224zc | 3-chloro-1,1,3,3-tétrafluoropropène |
| CHF₂-CF=CFCl | F-1224yb | 1-chloro-1,2,3,3-tétrafluoropropène |
| CF₃-CH=CFCl | F-1224zb | 1-chloro-1,3,3,3-tétrafluoropropène |
| CClF₂-CF=CHF | F-1224ye | 3-chloro-1,2,3,3-tétrafluoropropène |
| CF₃-CCl=CHF | F-1224xe | 2-chloro-1,3,3,3-tétrafluoropropène |
| CF₃-CF=CHCl | F-1224yd | 1-chloro-2,3,3,3-tétrafluoropropène |
| CF₃-CH=CF₂ | F-1225zc | 1,1,3,3,3-pentafluoropropène |
| CHF₂-CF=CF₂ | F-1225yc | 1,1,2,3,3-pentafluoropropène |
| CF₃-CF=CHF | F-1225ye | 1,2,3,3,3-pentafluoropropène |
| CH₂Cl-CCl=CCl₂ | F-1230xa | 1,1,2,3-tétrachloropropène |
| CHCl₂-CCl=CHCl | F-1230xd | 1,2,3,3-tétrachloropropène |
| CCl₃-CCl=CH₂ | F-1230xf | 2,3,3,3-tétrachloropropène |
| CHCl₂-CH=CCl₂ | F-1230za | 1,1,3,3-tétrachloropropène |
| CCl₃-CH=CHCl | F-1230zd | 1,3,3,3-tétrachloropropène |
| CF₃-CCl=CH₂ | F-1233xf | 2-chloro-3,3,3-trifluoropropène |
| CClF₂-CF=CH₂ | F-1233yf | 3-chloro-2,3,3-trifluoropropène |
| CHF₂-CF=CHCl | F-1233yd | 1-chloro-2,3,3-trifluoropropène |
| CF₃-CH=CHCl | F-1233zd | 1-chloro-3,3,3-trifluoropropène |
| CHF₂-CCl=CHF | F-1233xe | 2-chloro-1,3,3-trifluoropropène |
| CHClF-CF=CHF | F-1233ye | 3-chloro-1,2,3-trifluoropropène |
| CClF₂-CH=CHF | F-1233ze | 3-chloro-1,3,3-trifluoropropène |
| CH₂Cl-CF=CF₂ | F-1233yc | 3-chloro-1,1,2-trifluoropropène |
| CFH₂-CCl=CF₂ | F-1233xc | 2-chloro-1,1,3-trifluoropropène |
| CFClH-CH=CF₂ | F-1233zc | 3-chloro-1,1,3-trifluoropropène |
| CFH₂-CF=CFCl | F-1233yb | 1-chloro-1,2,3-trifluoropropène |
| CF₂H-CH=CFCl | F-1233zb | 1-chloro-1,3,3-trifluoropropène |
| CF₃-CF=CH₂ | F-1234yf | 2,3,3,3-tétrafluoropropène |
| CF₃-CH=CHF | F-1234ze | 1,3,3,3-tétrafluoropropène |
| CH₂F-CF=CF₂ | F-1234yc | 1,1,2,3-tétrafluoropropène |
| CHF₂-CH=CF₂ | F-1234zc | 1,1,3,3-tétrafluoropropène |
| CHF₂-CF=CHF | F-1234ye | 1,2,3,3-tétrafluoropropène |
| CH₃-CCl=CCl₂ | F-1240xa | 1,1,2-trichloropropène |
| CH₂Cl-CCl=CHCl | F-1240xd | 1,2,3-trichloropropène |
| CHCl₂-CCl=CH₂ | F-1240xf | 2,3,3-trichloropropène |
| CH₂Cl-CH=CCl₂ | F-1240za | 1,1,3-trichloropropène |
| CHCl₂-CH=CHCl | F-1240zd | 1,3,3-trichloropropène |
| CCl₃-CH=CH₂ | F-1240zf | 3,3,3-trichloropropène |
| CClF₂-CH=CH₂ | F-1242zf | 3-chloro-3,3-difluoropropène |
| CHClF-CF=CH₂ | F-1242yf | 3-chloro-2,3-difluoropropène |
| CHF₂-CCl=CH₂ | F-1242xf | 2-chloro-3,3-difluoropropène |
| CH₃-CCl=CF₂ | F-1242xc | 2-chloro-1,1-difluoropropène |
| CH₂Cl-CH=CF₂ | F-1242zc | 3-chloro-1,1-difluoropropène |
| CH₂Cl-CF=CHF | F-1242ye | 3-chloro-1,2-difluoropropène |
| CH₂F-CCl=CHF | F-1242xe | 2-chloro-1,3-difluoropropène |
| CHFCl-CH=CHF | F-1242ze | 3-chloro-1,3-difluoropropène |
| CH₂F-CF=CHCl | F-1242yd | 1-chloro-2,3-difluoropropène |
| CHF₂-CH=CHCl | F-1242zd | 1-chloro-3,3-difluoropropène |
| CH₂F-CH=CF₂ | F-1243zc | 1,1,3-trifluoropropène |
| CH₃-CF=CF₂ | F-1243yc | 1,1,2-trifluoropropène |
| CF₃-CH=CH₂ | F-1243zf | 3,3,3-trifluoropropène |
| CH₂F-CF=CHF | F-1243ye | 1,2,3-trifluoropropène |
| CHF₂-CF=CH₂ | F-1243yf | 2,3,3-trifluoropropène |
| CHF₂-CH=CHF | F-1243ze | 1,3,3-trifluoropropène |
| CH₃-CH=CCl₂ | F-1250za | 1,1-dichloropropène |
| CH₃-CCl=CHCl | F-1250xd | 1,2-dichloropropène |
| CH₂Cl-CCl=CH₂ | F-1250xf | 2,3-dichloropropène |
| CH₂Cl-CH=CHCl | F-1250zd | 1,3-dichloropropène |
| CHCl₂-CH=CH₂ | F-1250zf | 3,3-dichloropropène |
| CH₃-CH=CF₂ | F-1252zc | 1,1-difluoropropène |
| CH₃-CF=CHF | F-1252ye | 1,2-difluoropropène |
| CH₂F-CF=CH₂ | F-1252yf | 2,3-difluoropropène |
| CHF₂-CH=CH₂ | F-1252zf | 3,3-difluoropropène |
| CH₃-CCl=CH₂ | F-1260xf | 2-chloropropène |
| CH₃-CH=CHCl | F-1260zd | 1-chloropropène |
| CH₂Cl-CH=CH₂ | F-1260zf | 3-chloropropène |

Lorsque les composés ci-dessus existent sous la forme de deux isomères cis et trans, le nom du composé (par exemple le F-1234ze) désigne indifféremment l'une ou l'autre forme ou un mélange des deux formes, et les teneurs maximales indiquées sont alors des teneurs totales vis-à-vis des deux formes possibles - sauf lorsque la forme est précisée par la lettre E ou Z.

Par ailleurs, on désigne de manière générique par F-220 l'ensemble des composés heptachloropropanes, par F-230 l'ensemble des composés hexachloropropanes et ainsi de suite, en utilisant les notations du tableau ci-dessus sans les deux lettres finales.

### Compositions selon l'invention

L'invention propose des compositions à base de F-240fa. La teneur en F-240fa est supérieure ou égale à 99 %.

Selon certains modes de réalisation, elle est supérieure ou égale à 99,1 %, ou à 99,2 %, ou à 99,3 %, ou à 99,4 %, ou à 99,5 %, ou à 99,6 %, ou à 99,7 %, ou à 99,8 %, ou à 99,9 %, ou à 99,95 %.

Les compositions selon l'invention comprennent également au moins un composé choisi parmi une liste de composés supplémentaires qui est constituée par les séries F-220, F-230, F-250, F-260, F-270 ainsi que par les séries F-1210, F-1220, F-1230 (à l'exception du F-1230za et du F-1230zd, qui peuvent éventuellement être présents en quantités supérieures), F-1240, F-1250 et F-1260, ledit composé étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Ledit au moins un composé peut être présent en une teneur supérieure ou égale à 1 ppm, ou supérieure ou égale à 2 ppm, ou supérieure ou égale à 3 ppm, ou supérieure ou égale à 5 ppm.

Par exemple, ledit au moins un composé peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Un mode de réalisation concerne de telles compositions qui comprennent une pluralité (deux, trois, quatre ou plus de quatre) composés choisi parmi la liste de composés supplémentaires ci-dessus, la teneur de chacun desdits composés étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Chaque composé de cette pluralité peut alors être présent en une teneur supérieure ou égale à 1 ppm, ou supérieure ou égale à 2 ppm, ou supérieure ou égale à 3 ppm, ou supérieure ou égale à 5 ppm.

Par exemple, chaque composé de cette pluralité peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Un mode de réalisation concerne de telles compositions dans lesquelles la teneur de chacun des composés de la liste de composés supplémentaires ci-dessus éventuellement présent dans la composition est inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Chaque composé de la liste de composés supplémentaires peut alors être présent en une teneur supérieure ou égale à 1 ppm, ou supérieure ou égale à 2 ppm, ou supérieure ou égale à 3 ppm, ou supérieure ou égale à 5 ppm.

Par exemple, chaque composé de la liste de composés supplémentaires peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Les compositions selon l'invention peuvent notamment comprendre un ou plusieurs composés de la série F-220, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-220 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-220 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-220 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-220 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-220 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-230, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-230 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-230 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-230 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-230 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-230 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions peuvent comprendre un ou plusieurs composés de la série F-240, chacun (sauf le F-240fa) étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-240 (sauf F-240fa) dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-240 (sauf F-240fa) éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-240 (sauf F-240fa) dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-240 (sauf F-240fa) éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-240 (sauf F-240fa) dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Le F-240fa peut être présent en quantité nettement supérieure à celles listées ci-dessus.

Les compositions selon l'invention peuvent notamment comprendre un ou plusieurs composés de la série F-250, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-250 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-250 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-250 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-250 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-250 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-260, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-260 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-260 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-260 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-260 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-260 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-270, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-270 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-270 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-270 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-270 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-270 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre du F-1210xa, en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que le F-1210xa peut alors être présent en une teneur supérieure ou égale à 1 ppm, ou supérieure ou égale à 2 ppm, ou supérieure ou égale à 3 ppm, ou supérieure ou égale à 5 ppm.

Par exemple, le F-1210xa peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Les compositions selon l'invention peuvent notamment comprendre un ou plusieurs composés de la série F-1220, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-1220 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-1220 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1220 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-1220 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1220 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-1230 (à l'exception du F-1230za et du F-1230zd, qui peuvent éventuellement être présents en quantités supérieures), chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-1230 (sauf F-1230za et F-1230zd) dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-1230 (sauf F-1230za et F-1230zd) éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1230 (sauf F-1230za et F-1230zd) dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-1230 (sauf F-1230za et F-1230zd) éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1230 (sauf F-1230za et F-1230zd) dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Le F-1230za et le F-1230zd peuvent être présents en quantités nettement supérieures à celles listées ci-dessus. Ces composés sont des précurseurs du F-1234ze.

Les compositions selon l'invention peuvent notamment comprendre un ou plusieurs composés de la série F-1240, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-1240 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-1240 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1240 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-1240 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1240 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-1250, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-1250 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-1250 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1250 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-1250 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1250 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Les compositions selon l'invention peuvent notamment comprendre un ou plusieurs composés de la série F-1260, chacun étant présent dans la composition en une teneur inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm ; et la teneur totale en composés de la série F-1260 dans la composition, de préférence, étant inférieure ou égale à 500 ppm ; ou inférieure ou égale à 450 ppm ; ou inférieure ou égale à 400 ppm ; ou inférieure ou égale à 350 ppm ; ou inférieure ou égale à 300 ppm ; ou inférieure ou égale à 250 ppm ; ou inférieure ou égale à 200 ppm ; ou inférieure ou égale à 150 ppm ; ou inférieure ou égale à 100 ppm ; ou inférieure ou égale à 75 ppm ; ou inférieure ou égale à 50 ppm ; ou inférieure ou égale à 25 ppm ; ou inférieure ou égale à 10 ppm ; ou inférieure ou égale à 5 ppm.

Il est à noter que chaque composé de la série F-1260 éventuellement présent peut alors être présent en une teneur supérieure ou égale à 1 ppm, ou supérieure ou égale à 2 ppm, ou supérieure ou égale à 3 ppm, ou supérieure ou égale à 5 ppm. De même, la teneur totale en composés de la série F-1260 dans la composition peut être supérieure ou égale à 1 ppm, ou supérieure ou égale à 2 ppm, ou supérieure ou égale à 3 ppm, ou supérieure ou égale à 5 ppm.

Par exemple, chaque composé de la série F-1260 éventuellement présent peut être présent en une teneur de 1 à 5 ppm ; ou en une teneur de 5 à 10 ppm ; ou en une teneur de 10 à 25 ppm ; ou en une teneur de 25 à 50 ppm ; ou en une teneur de 50 à 75 ppm ; ou en une teneur de 75 à 100 ppm ; ou en une teneur de 100 à 150 ppm ; ou en une teneur de 150 à 200 ppm ; ou en une teneur de 200 à 250 ppm ; ou en une teneur de 250 à 300 ppm ; ou en une teneur de 300 à 350 ppm ; ou en une teneur de 350 à 400 ppm ; ou en une teneur de 400 à 450 ppm ; ou en une teneur de 450 à 500 ppm.

Par exemple, la teneur totale en composés de la série F-1260 dans la composition peut être de 1 à 5 ppm ; ou de 5 à 10 ppm ; ou de 10 à 25 ppm ; ou de 25 à 50 ppm ; ou de 50 à 75 ppm ; ou de 75 à 100 ppm ; ou de 100 à 150 ppm ; ou de 150 à 200 ppm ; ou de 200 à 250 ppm ; ou de 250 à 300 ppm ; ou de 300 à 350 ppm ; ou de 350 à 400 ppm ; ou de 400 à 450 ppm ; ou de 450 à 500 ppm.

Des impuretés particulièrement indésirables en mélange avec le F-1233zdE sont :
- les molécules de la série F-1215 et particulièrement le F-1215xc et le F-1215yc ;
- les molécules de la série F-1224 et particulièrement le F-1224yc, le F-1224zc et le F-1224ye ;
- les molécules de la série F-1233 différentes du F-1233zdE, et particulièrement le F-1233xf, le F-1233xc et le F-1233yc ;
- les molécules de la série F-1242 et particulièrement le F-1242zf.

Les molécules F-1215xc, F-1215yb et F-1215yc ont des points d'ébullition proches du F-1233zdE et sont donc difficiles à séparer de celui-ci. Du fait de leur réactivité, les molécules qui présentent un groupement =CF₂ ont également des risques d'effets toxicologiques. Cela concerne le F-1215xc et le F-1215yc parmi les molécules mentionnées ci-dessus.

Par conséquent, il est souhaitable d'ajuster les compositions selon l'invention de façon à limiter la présence de précurseurs de composés de la série F-1215 (et notamment de précurseurs du F-1215xc et du F-1215yc) dans celles-ci.

Des précurseurs possibles du F-1215xc et du F-1215yc par réaction de fluoration sont le F-1210xa, le F-220da (via le F-1210xa) et le F-220aa (via le F-1210xa).

Ainsi, des compositions avantageuses selon l'invention :
- comprennent au moins un composé parmi ceux des séries F-1210 et F-220, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1210 et F-220, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent au moins un composé parmi le F-1210xa, le F-220da et le F-220aa, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1210xa, le F-220da et le F-220aa, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

D'autres précurseurs possibles du F-1215xc et du F-1215yc par réaction de chlorofluoration (étant donné que le F-1233zdE peut être fabriqué par fluoration en présence de chlore en phase gazeuse) sont les composés des séries F-1220, F-1230, F-1240, F-1250 et F-1260.

Ainsi, des compositions avantageuses selon l'invention :
- comprennent au moins un composé parmi ceux des séries F-1220, F-1230 (sauf F-1230za et F-1230zd), F-1240, F-1250 et F-1260, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1220, F-1230 (sauf F-1230za et F-1230zd), F-1240, F-1250 et F-1260, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Encore d'autres compositions avantageuses selon l'invention :
- comprennent au moins un composé parmi ceux des séries F-1210, F-1220, F-1230 (sauf F-1230za et F-1230zd), F-1240, F-1250, F-1260 et F-220, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1210, F-1220, F-1230 (sauf F-1230za et F-1230zd), F-1240, F-1250, F-1260 et F-220, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Le F-1230za et le F-1230zd peuvent être présents en quantités nettement supérieures à celles listées ci-dessus.

Les molécules F-1224xe, F-1224yd, F-1224ye, F-1224zb et F-1224zc ont également des points d'ébullition proches du F-1233zdE et sont donc difficiles à séparer de celui-ci. Du fait de leur réactivité, les molécules qui présentent un groupement =CF₂ ont également des risques d'effets toxicologiques. Cela concerne le F-1224yc et le F-1224zc parmi les molécules mentionnées ci-dessus. En outre, le composé F-1224ye peut conduire, par fluoration successive, au composé F-1225ye, qui est également connu pour sa toxicité.

Par conséquent, il est souhaitable d'ajuster les compositions selon l'invention de façon à limiter la présence de précurseurs de composés de la série F-1224 (et notamment de précurseurs du F-1224yc, du F-1224zc et du F-1224ye) dans celles-ci.

Des précurseurs possibles du F-1224yc par réaction de fluoration sont le F-1220xa, le F-230aa (via le F-1220xa) et le F-230da (via le F-1220xa).

Des précurseurs possibles du F-1224zc par réaction de fluoration sont le F-1220za, le F-230fa (via le F-1220za) et le F-230da (via le F-1220za).

Des précurseurs possibles du F-1224ye par réaction de fluoration sont le F-1220xd, le F-230da (via le F-1220xd) et le F-230ab (via le F-1220xd).

Ainsi, des compositions avantageuses selon l'invention :
- comprennent au moins un composé parmi ceux des séries F-1220 et F-230, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1220 et F-230, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent au moins un composé parmi le F-1220xa, le F-1220za, le F-1220xd, le F-230aa, le F-230fa, le F-230ab et le F-230da, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1220xa, le F-1220za, le F-1220xd, le F-230aa, le F-230fa, le F-230ab et le F-230da, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

D'autres précurseurs possibles du F-1224yc, F-1224zc et du F-1224ye par réaction de chlorofluoration (étant donné que le F-1233zdE peut être fabriqué par fluoration en présence de chlore en phase gazeuse) sont les composés des séries F-1230, F-1240, F-1250 et F-1260.

Ainsi, des compositions avantageuses selon l'invention :
- comprennent au moins un composé parmi ceux des séries F-1230 (sauf F-1230za et F-1230zd), F-1240, F-1250 et F-1260, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1230 (sauf F-1230za et F-1230zd), F-1240, F-1250 et F-1260, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Encore d'autres compositions avantageuses selon l'invention :
- comprennent au moins un composé parmi ceux des séries F-1220, F-1230 (sauf F-1230za et F-1230zd), F-1240, F-1250, F-1260 et F-230, dans une teneur: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1220, F-1230 (sauf F-1230za et F-1230zd), F-1240, F-1250, F-1260 et F-230, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Le F-1230za et le F-1230zd peuvent être présents en quantités nettement supérieures à celles listées ci-dessus.

Les molécules de la série F-1233 présentent également des points d'ébullition proches de celui du F-1233zdE et sont donc difficiles à séparer de celui-ci.

Or le F-1233xf présente une aptitude élevée à la polymérisation et peut générer un polymère à longue chaîne qui peut ensuite se déposer sous forme de cristaux blancs. Il est donc préférable d'éviter la présence de cette impureté instable dans le composé final afin de faciliter l'utilisation du F-1233zdE dans l'application souhaitée.

De plus, les molécules F-1233xc et F-1233yc possèdent un groupement =CF2 et peuvent présenter un risque d'effet toxicologique.

Des précurseurs possibles du F-1233xf par réaction de fluoration sont le F-1230xf, le F-1230xa, le F-240db (via le F-1230xf et/ou le F-1230xa), le F-240ab (via le F-1230xf) et le F-240aa (via le F-1230xa).

Des précurseurs possibles du F-1233xc et du F-1233yc par réaction de fluoration sont le F-1230xa, le F-240db (via le F-1230xa) et le F-240aa (via le F-1230xa).

Ainsi, des compositions avantageuses selon l'invention :
- comprennent au moins un composé parmi ceux de la série F-1230 (sauf F-1230za et F-1230zd), dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1230 (sauf F-1230za et F-1230zd), la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent au moins un composé parmi le F-1230xf, le F-1230xa, dans une teneur: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1230xa, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Le F-240fa, le F-1230za et le F-1230zd peuvent être présents en quantités nettement supérieures à celles listées ci-dessus.

D'autres précurseurs possibles du F-1233xf, du F-1233yc et du F-1233xc par réaction de chlorofluoration (étant donné que le F-1233zdE peut être fabriqué par fluoration en présence de chlore en phase gazeuse) sont les composés des séries F-1240, F-1250 et F-1260.

Ainsi, encore d'autres compositions avantageuses selon l'invention :
- comprennent au moins un composé parmi ceux des séries F-1240, F-1250 et F-1260, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1230 (sauf F-1230za et F-1230zd), F-1240, F-1250, F-1260, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Le F-240fa, le F-1230za et le F-1230zd peuvent être présents en quantités nettement supérieures à celles listées ci-dessus.

La molécule F-1242zf présente également un point d'ébullition proche de celui du F-1233zdE et est donc difficile à séparer de celui-ci. Or ce composé est susceptible de former par fluoration successive le F-1243zf, molécule qui est indésirable en raison de sa toxicité.

Des précurseurs possibles du F-1242zf par réaction de fluoration sont le F-1240za, le F-1240zf, le F-250fb (via l'un des deux précédents), le F-250da (via le F-1240za) et le F-250db (via le F-1240zf).

Ainsi, des compositions avantageuses selon l'invention :
- comprennent au moins un composé parmi ceux des séries F-1240 et F-250, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1240 et F-250, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent au moins un composé parmi le F-1240za, le F-1240zf, le F-250fb, le F-250da et le F-250db, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi le F-1240za, le F-1240zf, le F-250fb, le F-250da et le F-250db, la teneur totale de tous ces composés étant : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

D'autres précurseurs possibles du F-1242zf par réaction de chlorofluoration (étant donné que le F-1233zdE peut être fabriqué par fluoration en présence de chlore en phase gazeuse) sont les composés des séries F-1250 et F-1260.

Ainsi, encore d'autres compositions avantageuses selon l'invention :
- comprennent au moins un composé parmi ceux des séries F-1250 et F-1260, dans une teneur : inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm ; ou bien
- comprennent un ou plusieurs composés parmi ceux des séries F-1240, F-1250, F-1260 et F-250, la teneur totale de tous ces composés étant: inférieure ou égale à 250 ppm ; ou de 150 à 200 ppm ; ou de 100 à 150 ppm ; ou de 50 à 100 ppm ; ou de 25 à 50 ppm ; ou de 10 à 25 ppm ; ou de 5 à 10 ppm ; ou inférieure ou égale à 5 ppm, et par exemple de 1 à 5 ppm.

Des impuretés particulièrement indésirables en mélange avec le F-1234zeE sont :
- les molécules de la série F-1216 et particulièrement le F-1216yc ;
- les molécules de la série F-1225 et particulièrement le F-1225ye et le F-1225zc ;
- les molécules de la série F-1243 et particulièrement le F-1243zf ; et
- les molécules de la série F-1234 différentes du F-1234zeE et particulièrement le F-1234yf.

Le F-1216yc est toxique et de point d'ébullition proche de celui du F-1234zeE, et donc difficile à séparer de celui-ci. Ses précurseurs par fluoration sont le F-1215xc et le F-1215yc. La manière d'éviter la présence de tels composés a déjà été décrite ci-dessus.

Le F-1225ye et le F-1225zc sont toxiques et de points d'ébullition proche de celui du F-1234zeE, et donc difficiles à séparer de celui-ci. Ses précurseurs par fluoration sont le F-1224ye et le F-1224zc. La manière d'éviter la présence de tels composés a déjà été décrite ci-dessus.

Le F-1243zf est toxique et de point d'ébullition proche de celui du F-1234zeE, et donc difficile à séparer de celui-ci. Son précurseur par fluoration est le F-1242zf. La manière d'éviter la présence de ce composé a déjà été décrite ci-dessus.

Le F-1234yf est une substance qui ne doit pas être présente en quantité trop élevée en mélange avec le F-1234zeE. Par exemple, sa teneur doit être inférieure ou égale à 500 ppm. Or les points d'ébullition des deux composés sont proches, ce qui rend une séparation conventionnelle difficile. Le précurseur du F-1234yf par fluoration est le F-1233xf. La manière d'éviter la présence de ce composé a déjà été décrite ci-dessus.

Il est à noter que la présence de molécules de la série F-270 peut être indésirable dans la mesure où, lorsqu'on effectue une réaction de fluoration en phase gazeuse en présence de chlore (pour la fabrication du F-1234zeE), ces molécules pourraient se chlorer et se fluorer pour produire certaines des impuretés indésirables énumérées ci-dessus.

### Préparation des compositions selon l'invention

La fabrication du F-240fa est connue par exemple du document US 5,705,779. Le document propose un procédé de production du F-240fa par :
- réaction du tétrachlorure de carbone avec de l'éthylène pour produire du F-250fb ;
- photochloration du F-250fb pour obtenir le F-240fa.

Les compositions selon l'invention peuvent ensuite être obtenues en procédant à une ou plusieurs étapes de séparation du F-240fa vis-à-vis des autres composés mentionnés ci-dessus, et notamment vis-à-vis du F-240db (qui est en général le sous-produit majoritaire de la chloration) et également des autres sous-produits de chloration comme les F-230 et/ou F-220 et/ou F-210.

Ces étapes de séparation peuvent de préférence être effectuées par absorption / lavage et distillation classique. Alternativement à la distillation classique ou en combinaison avec celle-ci, il est également possible de prévoir une séparation par distillation extractive, des séparations physico-chimiques sur tamis moléculaire, alumine ou charbon actif ou une séparation membranaire.

Une première séparation est généralement réalisée en utilisant une distillation standard (colonne à plateaux, colonne à garnissage) à pression atmosphérique ou sous pression réduite. La pression choisie est inférieure à 760 mmHg, préférentiellement inférieure à 450 mmHg et plus préférentiellement inférieure à 200 mmHg. De façon inhérente, la pression de la colonne détermine les conditions de température pour un degré de séparation choisi. Le F-240fa peut être récupéré en opérant la distillation à une température inférieure à 180°C, préférentiellement inférieure à 160°C et plus préférentiellement inférieure à 130°C. Une simple colonne ou un train de distillation peut être utilisé. Dans des conditions choisies, la pureté du F-240fa après distillation atteint au minimum 99,8 %.

Une seconde séparation peut être réalisée en utilisant une adsorption sur zéolite ou charbon actif.

Les zéolites ou charbons actifs utilisables dans le procédé de purification de F-240fa présentent avantageusement une taille moyenne de pores de 3,4 à 11 Å, de préférence de 3,4 à 10 Å. Si la zéolite ou le charbon actif a une taille de pores moyenne supérieure à 11 Å, la quantité de F-240fa adsorbée augmente, alors que si la taille moyenne des pores est inférieure à 3,4 Å, la capacité d'adsorption de la zéolite ou du charbon actif est réduite.

La zéolite a de préférence un rapport Si / Al de 2 ou moins. Si le rapport Si / Al de la zéolite est supérieur à deux, certaines impuretés sont susceptibles de ne pas être adsorbées sélectivement. La zéolite est de préférence au moins un élément choisi dans le groupe consistant en des tamis moléculaires 4A, un tamis moléculaire 5A, un tamis moléculaire 10X et des tamis moléculaires 13X. En utilisant ces zéolites, la teneur en eau dans le F-240fa peut également être réduite en même temps.

La zéolite et le charbon actif sont de préférence utilisés individuellement en vue de la régénération de l'adsorbant, mais ceux-ci peuvent également être utilisés en mélange. Les proportions de zéolite et de charbon actif dans le mélange ne sont pas particulièrement importantes, toutefois, il est préférable d'utiliser une quantité de zéolite plus élevée, ce qui permet de réduire la teneur en eau dans le F-240fa.

Pour traiter le F-240fa avec la zéolite et / ou le charbon actif en phase liquide, on peut utiliser un procédé discontinu (ou « batch ») ou un procédé continu. Industriellement, un procédé consistant à faire passer en continu le F-240fa sur un lit fixe est préférable. La vitesse spatiale horaire liquide (VSHL) peut être choisie de manière appropriée en fonction de la teneur en impuretés à éliminer et de la quantité de F-240fa à traiter. En général, la vitesse spatiale est de préférence de 1 à 50 h⁻¹. Industriellement le procédé de purification peut utiliser alternativement deux tours d'adsorption.

La température de traitement du F-240fa est de 0°C à 120°C, de préférence de 20°C à 80°C. Si la température de traitement est supérieure à 120°C, le coût de équipement peut augmenter en raison du chauffage de l'appareil, tandis que si la température de traitement est inférieure à 0°C un équipement de refroidissement peut être nécessaire. La pression est de 0 à 3 MPa, de préférence de 0 à 1 MPa. Si la pression est supérieure à 3 MPa, la rentabilité peut diminuer en raison des exigences en matière de résistance à la pression de l'appareil.

Une technique de séparation membranaire peut également être mise en oeuvre en complément d'une adsorption sur charbon actif ou sur zéolite, ou bien en alternative à ces techniques. La séparation membranaire peut être mise en oeuvre en phase gaz selon un procédé continu opéré à faible pression, ou à pression réduite. La pression choisie est inférieure à 5 bar, préférentiellement inférieure à 2 bar et plus préférentiellement inférieure à la pression atmosphérique. Le choix de la membrane dépend des propriétés des impuretés à séparer du F-240fa (différence de solubilité, de diffusivité et de perméabilité). La séparation membranaire est réalisée à une température dépendant de la pression choisie, inférieure à 250°C, préférentiellement inférieure à 230°C et plus préférentiellement inférieure à 180°C.

Lorsque le F-240fa contenant des impuretés est mis en contact avec la zéolite et / ou le charbon actif en phase liquide et / ou est purifié sur membrane en phase gazeuse dans les conditions décrites ci-dessus, le F-240db peut être obtenu avec une pureté supérieure à 99,9 %.

### Fabrication du F-1233zdE

Les compositions selon l'invention peuvent être utilisées pour la fabrication de F-1233zdE présentant des spécifications souhaitées, par une ou plusieurs étapes de fluoration.

La fluoration peut être une fluoration en phase liquide telle que décrite dans le document US 8,704,017.

Alternativement et de préférence, la fluoration est une fluoration catalytique en phase gazeuse par du HF en présence de chlore.

Le catalyseur utilisé peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32) et au document FR2748473 (en p.4), auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de chrome, et de 0,5 à 20 % en poids de nickel, de préférence de 2 à 10 % de chaque.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :
- avec un rapport molaire HF / composé chloré de 1:1 à 150:1, de préférence de 3:1 à 100:1 et de manière plus particulièrement préférée de 5:1 à 50:1 ;
- avec un rapport molaire de Cl₂ / composé chloré de 0,01:100 à 5:100, de préférence de 0,1:100 à 4:100 et de manière plus particulièrement préférée de 0,5:100 à 3:100 ;
- avec un temps de contact de 1 à 100 s, de préférence 1 à 50 s et plus particulièrement 2 à 40 s (volume de catalyseur divisé par le flux entrant total, ajusté à la température et à la pression de fonctionnement) ;
- à une pression absolue allant de 0,1 à 50 bar, de préférence de 0,3 à 15 bar ;
- à une température (température du lit de catalyseur) de 100 à 500°C, de préférence de 150 à 450°C, et plus particulièrement de 200 à 300°C.

Le flux de produits issu de la fluoration peut subir des traitements appropriés (distillation, lavage, etc.) afin de récupérer le F-1233zdE sous forme purifiée et le séparer des autres composés présents (HCl, HF non réagi, F-240fa non réagi, autres composés organiques). Un ou plusieurs flux peuvent faire l'objet d'un recyclage.

On peut également prévoir des étapes de régénération du catalyseur, comme décrit par exemple dans les documents WO 2012/098421 et WO 2012/098422 auxquels il est fait expressément référence.

Le flux de F-1233zdE obtenu, de préférence, contient :
- moins de 500 ppm, ou 250 ppm, ou 200 ppm, ou 150 ppm, ou 100 ppm, ou 50 ppm, ou 25 ppm, ou 10 ppm, ou 5 ppm, de F-1233xf ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1242zf ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1215yc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1215xc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1224yc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1224ye ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1224zc.

De préférence, ces teneurs sont obtenues à l'issue de la fluoration, sans (ou avant toute) étape de purification du flux de produit.

### Fabrication du F-1234zeE

Les compositions selon l'invention peuvent être utilisées pour la fabrication de F-1234zeE présentant des spécifications souhaitées, par une ou plusieurs étapes de fluoration à partir du F-1233zdE formé dans l'étape précédente.

La fluoration est de préférence une fluoration catalytique en phase gazeuse par du HF.

Le catalyseur utilisé peut être par exemple à base d'un métal comprenant un oxyde de métal de transition ou un dérivé ou un halogénure ou un oxyhalogénure d'un tel métal. On peut citer par exemple FeCl₃, l'oxyfluorure de chrome, les oxydes de chrome (éventuellement soumis à des traitements de fluoration), les fluorures de chrome et leurs mélanges. D'autres catalyseurs possibles sont les catalyseurs supportés sur du carbone, les catalyseurs à base d'antimoine, les catalyseurs à base d'aluminium (par exemple AlF₃ et Al₂O₃, l'oxyfluorure d'alumine et le fluorure d'alumine).

On peut utiliser en général un oxyfluorure de chrome, un fluorure ou un oxyfluorure d'aluminium, ou un catalyseur supporté ou non contenant un métal tel que Cr, Ni, Fe, Zn, Ti, V, Zr, Mo, Ge, Sn, Pb, Mg, Sb.

On peut faire référence à cet égard au document WO 2007/079431 (en p.7, I.1-5 et 28-32), au document US 5,895,825 auxquels il est fait expressément référence.

Le catalyseur est de manière plus particulièrement préférée à base de chrome et il s'agit plus particulièrement d'un catalyseur mixte comprenant du chrome.

Selon un mode de réalisation, on utilise un catalyseur mixte comprenant du chrome et du nickel. Le rapport molaire Cr / Ni (sur la base de l'élément métallique) est généralement de 0,5 à 5, par exemple de 0,7 à 2, par exemple d'environ 1. Le catalyseur peut contenir de 0,5 à 20 % en poids de chrome, et de 0,5 à 20 % en poids de nickel, de préférence de 2 à 10 % de chaque.

Le métal peut être présent sous forme métallique ou sous forme de dérivé, par exemple un oxyde, halogénure ou oxyhalogénure. Ces dérivés sont de préférence obtenus par activation du métal catalytique.

Le support est de préférence constitué avec de l'aluminium, par exemple de l'alumine, de l'alumine activée ou des dérivés d'aluminium, tels que les halogénures d'aluminium et les oxyhalogénures d'aluminium, par exemple décrits dans le document US 4,902,838, ou obtenus par le procédé d'activation décrit ci-dessus.

Le catalyseur peut comprendre du chrome et du nickel sous une forme activée ou non, sur un support qui a été soumis à une activation ou non.

On peut se reporter au document WO 2009/118628 (notamment en p.4, I.30-p.7 I.16), auquel il est fait expressément référence ici.

Un autre mode de réalisation préféré repose sur un catalyseur mixte contenant du chrome et au moins un élément choisi parmi Mg et Zn. Le rapport atomique de Mg ou Zn/Cr est de préférence de 0,01 à 5.

Avant son utilisation, le catalyseur est de préférence soumis à une activation avec de l'air, de l'oxygène ou du chlore et/ou avec de l'HF.

Par exemple, le catalyseur est de préférence soumis à une activation avec de l'air ou de l'oxygène et du HF à une température de 100 à 500°C, de préférence de 250 à 500°C et plus particulièrement de 300 à 400°C. La durée d'activation est de préférence de 1 à 200 h et plus particulièrement de 1 à 50 h.

Cette activation peut être suivie d'une étape d'activation de fluoration finale en présence d'un agent d'oxydation, d'HF et de composés organiques.

Le rapport molaire HF / composés organiques est de préférence de 2 à 40 et le rapport molaire agent d'oxydation / composés organiques est de préférence de 0,04 à 25. La température de l'activation finale est de préférence de 300 à 400°C et sa durée de préférence de 6 à 100 h.

La réaction de fluoration en phase gazeuse peut être effectuée :
- avec un rapport molaire HF / composé chloré de 1:1 à 150:1, de préférence de 1.5:1 à 100:1 et de manière plus particulièrement préférée de 2:1 à 50:1 ;
- avec un temps de contact de 1 à 100 s, de préférence 1 à 50 s et plus particulièrement 2 à 40 s (volume de catalyseur divisé par le flux entrant total, ajusté à la température et à la pression de fonctionnement) ;
- à une pression absolue allant de 0,1 à 50 bar, de préférence de 0,3 à 15 bar ;
- à une température (température du lit de catalyseur) de 100 à 500°C, de préférence de 200 à 450°C, et plus particulièrement de 250 à 400°C.

La durée de l'étape de réaction est typiquement de 10 à 2000 heures, de préférence de 50 à 500 heures et de manière plus particulièrement préférée de 70 à 300 heures.

Un agent oxydant, de préférence l'oxygène, peut éventuellement être ajouté lors de la réaction de fluoration. Le rapport molaire oxygène / composés organiques peut être de 0,005 à 2, de préférence de 0,01 à 1,5. L'oxygène peut être introduit pur ou sous forme d'air ou de mélange oxygène / azote. On peut également remplacer l'oxygène par du chlore.

Le flux de produits issu de la fluoration peut subir des traitements appropriés (distillation, lavage, etc.) afin de récupérer le F-1234zeE sous forme purifiée et le séparer des autres composés présents (HCl, HF non réagi, F-240fa non réagi, autres composés organiques). Un ou plusieurs flux peuvent faire l'objet d'un recyclage.

On peut également prévoir des étapes de régénération du catalyseur, comme décrit par exemple dans les documents WO 2012/098421 et WO 2012/098422 auxquels il est fait expressément référence.

Le flux de F-1234zeE obtenu, de préférence, contient :
- moins de 500 ppm, ou 250 ppm, ou 200 ppm, ou 150 ppm, ou 100 ppm, ou 50 ppm, ou 25 ppm, ou 10 ppm, ou 5 ppm, de F-1243zf ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1225zc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1216yc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1243yc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1252zc ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1225ye ; et/ou
- moins de 500 ppm, ou 250 ppm, ou 200 ppm, ou 150 ppm, ou 100 ppm, ou 50 ppm, ou 25 ppm, ou 10 ppm, ou 5 ppm, de F-1234yf.

De préférence, ces teneurs sont obtenues à l'issue de la fluoration, sans (ou avant toute) étape de purification du flux de produit.

### EXEMPLES

Les exemples suivants illustrent l'invention sans la limiter.

### Exemple 1 : analyse de deux compositions à base de F-240fa

On considère deux compositions A et B à base de F-240fa, de puretés différentes. La première composition A est issue d'une synthèse et d'une purification dans les laboratoires de la demanderesse. La deuxième composition B provient d'un fournisseur commercial, Synquest Laboratories.

Les compositions en mol% de ces deux échantillons sont données dans le tableau 1 ci-dessous (après analyse chromatographique en phase gazeuse).

**Tableau 1 - Analyse des compositions A et B**

| | Composition A | Composition B |
|---|---|---|
| F-1230za | 0,055 | 0,018 |
| F-250 | 0,035 | 0,449 |
| F-240fa | 99,58 | 96,79 |
| C₂Cl₆ | 0,051 | 0,239 |
| F-240db | 0,157 | 2,46 |
| Autres | 0,122 | 0,044 |

### Exemple 2 : Préparation du catalyseur de fluoration

Dans un évaporateur rotatif, on place 343 g d'un support d'alumine Grace HSA préalablement prétraité en lit fixe à 280°C sous un mélange HF / air contenant entre 5 et 10 % d'HF. L'alumine de départ se présente sous la forme de billes de diamètre compris entre 0,5 et 2 mm. Sa surface spécifique est environ de 220 m²/g et son volume poreux de 1,3 cm³/g. Par ailleurs, on prépare deux solutions aqueuses :
- l'une contient 81 g de méthanol et 8 g d'eau ;
- l'autre contient 62 g d'eau, 55 g d'acide chromique CrO₃ et 130 g de chlorure de nickel NiCl₂ (dissolution du mélange à 50°C pendant 2h30).

Les deux solutions sont introduites simultanément et progressivement sur le support d'alumine maintenu à 40°C et agité. Après une étape de maturation sous azote, le catalyseur est séché sous azote, puis sous vide à 65°C, puis vers 90°C pendant 6 heures.

On charge 500 g de solide imprégné dans un réacteur tubulaire en inconel. Le catalyseur est tout d'abord séché sous balayage d'azote à 320°C, à pression atmosphérique. Il est ensuite fluoré en présence d'un mélange HF/N₂ (5 à 10% d'HF dans l'azote) à 320°C puis jusqu'à 390°C. L'alimentation en HF est ensuite coupée. Le catalyseur est refroidi sous azote.

### Exemple 3 : Fluoration en phase gazeuse

L'exemple a été réalisé à l'aide d'un pilote de fluoration en phase gaz en continu. Ce pilote comprend un réacteur constitué d'un tube en inconel d'un diamètre interne de 38 mm et d'une longueur de 500 mm, placé verticalement dans un four électrique tubulaire. Un puits thermométrique de diamètre extérieur de 6 mm est placé coaxialement dans le four et permet une lecture de température le long du lit catalytique à l'aide de quatre thermocouples étagés.

Un serpentin enroulé autour du réacteur et traversé verticalement de bas en haut permet de préchauffer les réactifs avant l'entrée dans le réacteur. Une couche de corindon de 30 mL au-dessus du lit catalytique permet d'assurer une distribution homogène des réactifs gazeux. Une vanne de régulation permet de maintenir la pression souhaitée. Le flux gazeux à l'entrée et la sortie du réacteur est analysé par chromatographie phase gaz.

Une quantité adéquate de solide décrit ci-dessus est introduite dans le réacteur, puis séchée pendant une nuit en présence d'azote à 250° C et pression atmosphérique. Le solide séché est ensuite activé (toujours à pression atmosphérique) sous un flux comprenant l'azote et l'acide fluorhydrique anhydre pendant 15 minutes avant d'être placé sous HF pur à 250°C. La pression est ensuite régulée très progressivement jusqu'à atteindre 10 bars absolus. Les réactifs (le chlore et la composition A) sont ensuite introduits. Les débits alimentés sont tels que le rapport molaire HF / F-240fa est égal à 20, le rapport molaire Cl₂ / F-240fa est égal à 0,018, le temps de contact est de 15 secondes. La température est maintenue à 250°C. La composition du flux gazeux sortant du réacteur est analysée en chromatographie phase gazeuse et est reportée dans le tableau 1.

L'expérimentation est également reproduite dans les mêmes conditions opératoires avec l'échantillon B de F-240fa contenant 2,46 % de F-240db. La composition du flux gazeux sortant du réacteur est analysée en chromatographie phase gazeuse et est reportée dans le tableau 2 ci-dessous en mol%.

**Tableau 2 - Fluoration en phase gazeuse de compositions à base de F-240fa la production de F-1233zdE**

| | Réaction avec la composition A | Réaction avec la composition B |
|---|---|---|
| F-1233zdE | 80,2 | 78,6 |
| F-1233zdZ | 12,8 | 11,7 |
| F-243fa | 0,7 | 0,5 |
| F-244fa | 2,6 | 3,1 |
| F-245fa | 1,4 | 2 |
| F-1233xf | 0,14 | 2,12 |
| F-1234zeE | 1,9 | 1,81 |
| F-1234zeZ | 0,1 | 0,09 |
| F-1232zd | 0,09 | 0,03 |
| F-1232za | 0,07 | 0,05 |

### Exemple 4 : mise en évidence de la polymérisation du F-1233xf

On dispose d'un autoclave de 100 mL équipé d'une mesure de température et d'une mesure de pression. Celui-ci est plongé dans un bain d'huile dont la température est régulée. On introduit 49,2 g de composé F-1233xf de pureté 99,67% dans l'autoclave et la température du réacteur est augmentée à 56°C. La pression relative autogène est alors de 2,8 bars. Le composé est laissé en température pendant 18 heures. A l'issue de cette période, le réacteur est ramené à température ambiante puis dépressurisé vers un piège inox refroidi dans l'azote liquide. Le piège froid est ensuite détendu puis analysé : on obtient 99,67% de F-1233xf. La composition du produit récupéré après dégazage est identique à la composition du produit de départ. On note l'aspect visuel du fond de réacteur où un film huileux s'est déposé. L'autoclave est ensuite rincé à l'aide d'une solution de dichlorométhane qui est analysée en chromatographie phase liquide. L'analyse révèle la présence de 1100 ppm d'un composé identifié par la technique spectrométrie de masse couplée à la chromatographie : C₉F₉H₆Cl₃, c'est-à-dire le trimère du composé F-1233xf.

### Exemple 5 : mise en évidence de la polymérisation du F-1233xf en milieu acide

L'exemple 4 est reproduit avec 12,1 g de F-1233xf mis en présence de 41,6g de HF. Le mélange est laissé pendant 18 heures sous une température de 79°C et une pression autogène relative de 7,6 bars. Le composé collecté dans le piège froid après dépressurisation, lavage dans un barboteur et séchage présente toujours une pureté de 99,67%. Dans ces conditions opératoires, le fond du réacteur est recouvert de cristaux blancs. Environ 1g de ces cristaux a pu être récupéré. Des analyses en Infra-rouge et en RMN ont permis d'identifier un composé oligopolymérique constitué de groupements (-CClCF₃-CH₂-)n.

## Revendications

1. Composition comprenant au moins 99 % en poids de 1,1,1,3,3-pentachloropropane, et comprenant au moins un composé choisi parmi une liste de composés supplémentaires consistant en les dichloropropanes, les trichloropropanes, les tétrachloropropanes, les hexachloropropanes, les heptachloropropanes, les chloropropènes, les dichloropropènes, les trichloropropènes, les tétrachloropropènes, les pentachloropropènes et l'hexachloropropène, ledit composé étant présent dans la composition en une teneur pondérale inférieure ou égale à 500 ppm.

2. Composition selon la revendication 1, dans laquelle ledit composé est présent dans la composition en une teneur pondérale inférieure ou égale à 250 ppm ; de préférence inférieure ou égale à 150 ppm ; plus particulièrement inférieure ou égale à 100 ppm ; plus particulièrement inférieure ou égale à 50 ppm ; et idéalement inférieure ou égale à 10 ppm.

3. Composition selon l'une des revendications 1 à 2, comprenant une pluralité de composés choisis parmi ladite liste de composés supplémentaires, chacun des composés de ladite pluralité de composés étant présent dans la composition en une teneur pondérale inférieure ou égale à 500 ppm ; de préférence inférieure ou égale à 250 ppm ; de préférence inférieure ou égale à 150 ppm ; plus particulièrement inférieure ou égale à 100 ppm ; plus particulièrement inférieure ou égale à 50 ppm ; et idéalement inférieure ou égale à 10 ppm.

4. Composition selon l'une des revendications 1 à 3, comprenant une pluralité de composés choisis parmi ladite liste de composés supplémentaires, la teneur pondérale totale de l'ensemble des composés de ladite liste étant inférieure ou égale à 1000 ppm ; de préférence inférieure ou égale à 500 ppm ; de préférence inférieure ou égale à 250 ppm ; de préférence inférieure ou égale à 150 ppm ; plus particulièrement inférieure ou égale à 100 ppm ; plus particulièrement inférieure ou égale à 50 ppm ; et idéalement inférieure ou égale à 10 ppm.

5. Composition selon l'une des revendications 1 à 4, comprenant au moins 99,5 % en poids, et de préférence au moins 99,8 % en poids, et de manière plus particulièrement préférée au moins 99,9 % en poids, de 1,1,1,3,3-pentachloropropane.

6. Composition selon l'une des revendications 1 à 5, comprenant au moins un composé choisi parmi le groupe constitué de l'hexachloropropène et des heptachloropropanes, et dans laquelle la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et dans laquelle, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

7. Composition selon l'une des revendications 1 à 6, comprenant au moins un composé choisi parmi le groupe constitué des pentachloropropènes et des hexachloropropanes, et dans laquelle la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et dans laquelle, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

8. Composition selon l'une des revendications 1 à 7, comprenant au moins un composé choisi parmi le groupe constitué des tétrachloropropènes, et dans laquelle la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et dans laquelle, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

9. Composition selon l'une des revendications 1 à 8, comprenant au moins un composé choisi parmi le groupe constitué du 2,3,3,3-tétrachloropropène, du 1,1,2,3-tétrachloropropène, dans laquelle la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et dans laquelle, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

10. Composition selon l'une des revendications 1 à 9, comprenant au moins un composé choisi parmi le groupe constitué des trichloropropènes et des tétrachloropropanes, et dans laquelle la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et dans laquelle, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

11. Composition selon l'une des revendications 1 à 10, comprenant au moins un composé choisi parmi le groupe constitué du 1,1,3-trichloropropène, du 3,3,3-trichloropropène, du 1,1,1,3-tétrachloropropane, du 1,1,2,3-tétrachloropropane et du 1,1,1,2-tétrachloropropane, et dans laquelle la teneur pondérale de chacun de ces composés dans la composition est inférieure ou égale à 500 ppm, de préférence inférieure ou égale à 200 ppm, plus particulièrement inférieure ou égale à 100 ppm et idéalement inférieure ou égale à 50 ppm ; et dans laquelle, optionnellement, la teneur pondérale totale des composés de ce groupe dans la composition est inférieure ou égale à 500 ppm, de préférence inférieure ou égale à 200 ppm, plus particulièrement inférieure ou égale à 100 ppm et idéalement inférieure ou égale à 50 ppm.

12. Procédé de production de 1,3,3,3-tétrafluoropropène, notamment sous forme trans, comprenant :
- la fourniture d'une composition selon l'une des revendications 1 à 11 ;
- la réaction de cette composition avec de l'acide fluorhydrique, de préférence en phase gazeuse.

13. Procédé selon la revendication 12, comprenant une unique étape de fluoration catalytique.

14. Procédé selon la revendication 12, comprenant deux étapes successives de fluoration catalytique, à savoir :
- la réaction de la composition avec de l'acide fluorhydrique en phase gazeuse, pour fabriquer un produit intermédiaire ;
- optionnellement, une purification du produit intermédiaire ; puis
- la réaction du produit intermédiaire avec de l'acide fluorhydrique en phase gazeuse, pour fabriquer le 1,3,3,3-tétrafluoropropène ;
le produit intermédiaire étant de préférence le 1-chloro-3,3,3-trifluoropropène, notamment sous forme trans.

## Patentansprüche

1. Zusammensetzung, umfassend mindestens 99 Gew.-% 1,1,1,3,3-Pentachlorpropan und mindestens eine Verbindung aus einer Liste zusätzlicher Verbindungen bestehend aus Dichlorpropanen, Trichlorpropanen, Tetrachlorpropanen, Hexachlorpropanen, Heptachlorpropanen, Chlorpropenen, Dichlorpropenen, Trichlorpropenen, Tetrachlorpropenen, Pentachlorpropenen und Hexachlorpropen, wobei die Verbindung in der Zusammensetzung in einem Gewichtsgehalt kleiner oder gleich 500 ppm vorliegt.

2. Zusammensetzung nach Anspruch 1, wobei die Verbindung in der Zusammensetzung in einem Gewichtsgehalt kleiner oder gleich 250 ppm, vorzugsweise kleiner oder gleich 150 ppm, spezieller kleiner oder gleich 100 ppm, spezieller kleiner oder gleich 50 ppm und idealerweise kleiner oder gleich 10 ppm vorliegt.

3. Zusammensetzung nach Anspruch 1 oder 2, umfassend mehrere Verbindungen aus der Liste zusätzlicher Verbindungen, wobei jede der Verbindungen der mehreren Verbindungen in der Zusammensetzung in einem Gewichtsgehalt kleiner oder gleich 500 ppm, vorzugsweise kleiner oder gleich 250 ppm, vorzugsweise kleiner oder gleich 150 ppm, spezieller kleiner oder gleich 100 ppm, spezieller kleiner oder gleich 50 ppm und idealerweise kleiner oder gleich 10 ppm vorliegt.

4. Zusammensetzung nach einem der Ansprüche 1 bis 3, umfassend mehrere Verbindungen aus der Liste zusätzlicher Verbindungen, wobei der gesamte Gewichtsgehalt aller Zusammensetzungen der Liste kleiner oder gleich 1000 ppm, vorzugsweise kleiner oder gleich 500 ppm, vorzugsweise kleiner oder gleich 250 ppm, vorzugsweise kleiner oder gleich 150 ppm, spezieller kleiner oder gleich 100 ppm, spezieller kleiner oder gleich 50 ppm und idealerweise kleiner oder gleich 10 ppm ist.

5. Zusammensetzung nach einem der Ansprüche 1 bis 4, die mindestens 99,5 Gew.-% und vorzugsweise mindestens 99,8 Gew.-% und spezieller bevorzugt mindestens 99,9 Gew.-% 1,1,1,3,3-Pentachlorpropan umfasst.

6. Zusammensetzung nach einem der Ansprüche 1 bis 5, umfassend mindestens eine Verbindung, die aus der Gruppe bestehend aus Hexachlorpropen und Heptachlorpropanen ausgewählt ist, wobei der Gewichtsgehalt jeder dieser Verbindungen in der Zusammensetzung kleiner oder gleich 500 ppm, vorzugsweise kleiner oder gleich 200 ppm, weiter bevorzugt kleiner gleich 100 ppm und idealerweise kleiner oder gleich 50 ppm ist und wobei gegebenenfalls der gesamte Gewichtsgehalt der Verbindungen dieser Gruppe in der Zusammensetzung kleiner oder gleich 500 ppm, vorzugsweise kleiner oder gleich 200 ppm, spezieller kleiner oder gleich 100 ppm und idealerweise kleiner oder gleich 50 ppm ist.

7. Zusammensetzung nach einem der Ansprüche 1 bis 6, umfassend mindestens eine Verbindung, die aus der Gruppe bestehend aus Pentachlorpropenen und Hexachlorpropanen ausgewählt ist, wobei der Gewichtsgehalt jeder dieser Verbindungen in der Zusammensetzung kleiner oder gleich 500 ppm, vorzugsweise kleiner oder gleich 200 ppm, weiter bevorzugt kleiner gleich 100 ppm und idealerweise kleiner oder gleich 50 ppm ist und wobei gegebenenfalls der gesamte Gewichtsgehalt der Verbindungen dieser Gruppe in der Zusammensetzung kleiner oder gleich 500 ppm, vorzugsweise kleiner oder gleich 200 ppm, spezieller kleiner oder gleich 100 ppm und idealerweise kleiner oder gleich 50 ppm ist.

8. Zusammensetzung nach einem der Ansprüche 1 bis 7, umfassend mindestens eine Verbindung, die aus der Gruppe bestehend aus Tetrachlorpropenen ausgewählt ist, wobei der Gewichtsgehalt jeder dieser Verbindungen in der Zusammensetzung kleiner oder gleich 500 ppm, vorzugsweise kleiner oder gleich 200 ppm, weiter bevorzugt kleiner gleich 100 ppm und idealerweise kleiner oder gleich 50 ppm ist und wobei gegebenenfalls der gesamte Gewichtsgehalt der Verbindungen dieser Gruppe in der Zusammensetzung kleiner oder gleich 500 ppm, vorzugsweise kleiner oder gleich 200 ppm, spezieller kleiner oder gleich 100 ppm und idealerweise kleiner oder gleich 50 ppm ist.

9. Zusammensetzung nach einem der Ansprüche 1 bis 8, umfassend mindestens eine Verbindung, die aus der Gruppe bestehend aus 2,3,3,3-Tetrachlorpropen und 1,1,2,3-Tetrachlorpropen ausgewählt ist, wobei der Gewichtsgehalt jeder dieser Verbindungen in der Zusammensetzung kleiner oder gleich 500 ppm, vorzugsweise kleiner oder gleich 200 ppm, weiter bevorzugt kleiner gleich 100 ppm und idealerweise kleiner oder gleich 50 ppm ist und wobei gegebenenfalls der gesamte Gewichtsgehalt der Verbindungen dieser Gruppe in der Zusammensetzung kleiner oder gleich 500 ppm, vorzugsweise kleiner oder gleich 200 ppm, spezieller kleiner oder gleich 100 ppm und idealerweise kleiner oder gleich 50 ppm ist.

10. Zusammensetzung nach einem der Ansprüche 1 bis 9, umfassend mindestens eine Verbindung, die aus der Gruppe bestehend aus Trichlorpropenen und Tetrachlorpropanen ausgewählt ist, wobei der Gewichtsgehalt jeder dieser Verbindungen in der Zusammensetzung kleiner oder gleich 500 ppm, vorzugsweise kleiner oder gleich 200 ppm, weiter bevorzugt kleiner gleich 100 ppm und idealerweise kleiner oder gleich 50 ppm ist und wobei gegebenenfalls der gesamte Gewichtsgehalt der Verbindungen dieser Gruppe in der Zusammensetzung kleiner oder gleich 500 ppm, vorzugsweise kleiner oder gleich 200 ppm, spezieller kleiner oder gleich 100 ppm und idealerweise kleiner oder gleich 50 ppm ist.

11. Zusammensetzung nach einem der Ansprüche 1 bis 10, umfassend mindestens eine Verbindung, die aus der Gruppe bestehend aus 1,1,3-Trichlorpropen, 3,3,3-Trichlorpropen, 1,1,1,3-Tetrachlorpropan, 1,1,2,3-Tetrachlorpropan und 1,1,1,2-Tetrachlorpropan ausgewählt ist, wobei der Gewichtsgehalt jeder dieser Verbindungen in der Zusammensetzung kleiner oder gleich 500 ppm, vorzugsweise kleiner oder gleich 200 ppm, weiter bevorzugt kleiner gleich 100 ppm und idealerweise kleiner oder gleich 50 ppm ist und wobei gegebenenfalls der gesamte Gewichtsgehalt der Verbindungen dieser Gruppe in der Zusammensetzung kleiner oder gleich 500 ppm, vorzugsweise kleiner oder gleich 200 ppm, spezieller kleiner oder gleich 100 ppm und idealerweise kleiner oder gleich 50 ppm ist.

12. Verfahren zur Herstellung von 1,3,3,3-Tetrafluorpropen, insbesondere in trans-Form, umfassend:
- Bereitstellung einer Zusammensetzung nach einem der Ansprüche 1 bis 11;
- Umsetzung dieser Zusammensetzung mit Fluorwasserstoffsäure in der Gasphase.

13. Verfahren nach Anspruch 12, das einen einzigen Schritt der katalytischen Fluorierung umfasst.

14. Verfahren nach Anspruch 12, das zwei aufeinanderfolgende Schritte der katalytischen Fluorierung umfasst, nämlich:
- Umsetzung der Zusammensetzung mit Fluorwasserstoffsäure in der Gasphase zur Herstellung eines Zwischenprodukts;
- gegebenenfalls eine Reinigung des Zwischenprodukts; dann
- Umsetzung des Zwischenprodukts mit Fluorwasserstoffsäure in der Gasphase zur Herstellung von 1,3,3,3-Tetrafluorpropen;
wobei es sich bei dem Zwischenprodukt vorzugsweise um 1-Chlor-3,3,3-trifluorpropen, insbesondere in trans-Form, handelt.

## Claims

1. Composition comprising at least 99% by weight of 1,1,1,3,3-pentachloropropane, and comprising at least one compound chosen from a list of additional compounds consisting of dichloropropanes, trichloropropanes, tetrachloropropanes, hexachloropropanes, heptachloropropanes, chloropropenes, dichloropropenes, trichloropropenes, tetrachloropropenes, pentachloropropenes and hexachloropropene, said compound being present in the composition in a weight content of less than or equal to 500 ppm.

2. Composition according to Claim 1, in which said compound is present in the composition in a weight content of less than or equal to 250 ppm; preferably less than or equal to 150 ppm; more particularly less than or equal to 100 ppm; more particularly less than or equal to 50 ppm; and ideally less than or equal to 10 ppm.

3. Composition according to either of Claims 1 and 2, comprising a plurality of compounds chosen from said list of additional compounds, each of the compounds of said plurality of compounds being present in the composition in a weight content of less than or equal to 500 ppm; preferably less than or equal to 250 ppm; preferably less than or equal to 150 ppm; more particularly less than or equal to 100 ppm; more particularly less than or equal to 50 ppm; and ideally less than or equal to 10 ppm.

4. Composition according to one of Claims 1 to 3, comprising a plurality of compounds chosen from said list of additional compounds, the total weight content of all of the compounds of said list being less than or equal to 1000 ppm; preferably less than or equal to 500 ppm; preferably less than or equal to 250 ppm; preferably less than or equal to 150 ppm; more particularly less than or equal to 100 ppm; more particularly less than or equal to 50 ppm; and ideally less than or equal to 10 ppm.

5. Composition according to one of Claims 1 to 4, comprising at least 99.5% by weight, preferably at least 99.8% by weight, and more particularly preferably at least 99.9% by weight, of 1,1,1,3,3-pentachloropropane.

6. Composition according to one of Claims 1 to 5, comprising at least one compound chosen from the group consisting of hexachloropropene and heptachloropropanes, and in which the weight content of each of these compounds in the composition is less than or equal to 500 ppm, preferably less than or equal to 200 ppm, more particularly less than or equal to 100 ppm and ideally less than or equal to 50 ppm; and in which, optionally, the total weight content of the compounds of this group in the composition is less than or equal to 500 ppm, preferably less than or equal to 200 ppm, more particularly less than or equal to 100 ppm and ideally less than or equal to 50 ppm.

7. Composition according to one of Claims 1 to 6, comprising at least one compound chosen from the group consisting of pentachloropropenes and hexachloropropanes, and in which the weight content of each of these compounds in the composition is less than or equal to 500 ppm, preferably less than or equal to 200 ppm, more particularly less than or equal to 100 ppm and ideally less than or equal to 50 ppm; and in which, optionally, the total weight content of the compounds of this group in the composition is less than or equal to 500 ppm, preferably less than or equal to 200 ppm, more particularly less than or equal to 100 ppm and ideally less than or equal to 50 ppm.

8. Composition according to one of Claims 1 to 7, comprising at least one compound chosen from the group consisting of tetrachloropropenes, and in which the weight content of each of these compounds in the composition is less than or equal to 500 ppm, preferably less than or equal to 200 ppm, more particularly less than or equal to 100 ppm and ideally less than or equal to 50 ppm; and in which, optionally, the total weight content of the compounds of this group in the composition is less than or equal to 500 ppm, preferably less than or equal to 200 ppm, more particularly less than or equal to 100 ppm and ideally less than or equal to 50 ppm.

9. Composition according to one of Claims 1 to 8, comprising at least one compound chosen from the group consisting of 2,3,3,3-tetrachloropropene, 1,1,2,3-tetrachloropropene, in which the weight content of each of these compounds in the composition is less than or equal to 500 ppm, preferably less than or equal to 200 ppm, more particularly less than or equal to 100 ppm and ideally less than or equal to 50 ppm; and in which, optionally, the total weight content of the compounds of this group in the composition is less than or equal to 500 ppm, preferably less than or equal to 200 ppm, more particularly less than or equal to 100 ppm and ideally less than or equal to 50 ppm.

10. Composition according to one of Claims 1 to 9, comprising at least one compound chosen from the group consisting of trichloropropenes and tetrachloropropanes, and in which the weight content of each of these compounds in the composition is less than or equal to 500 ppm, preferably less than or equal to 200 ppm, more particularly less than or equal to 100 ppm and ideally less than or equal to 50 ppm; and in which, optionally, the total weight content of the compounds of this group in the composition is less than or equal to 500 ppm, preferably less than or equal to 200 ppm, more particularly less than or equal to 100 ppm and ideally less than or equal to 50 ppm.

11. Composition according to one of Claims 1 to 10, comprising at least one compound chosen from the group consisting of 1,1,3-trichloropropene, 3,3,3-trichloropropene, 1,1,1,3-tetrachloropropane, 1,1,2,3-tetrachloropropane and 1,1,1,2-tetrachloropropane, and in which the weight content of each of these compounds in the composition is less than or equal to 500 ppm, preferably less than or equal to 200 ppm, more particularly less than or equal to 100 ppm and ideally less than or equal to 50 ppm; and in which, optionally, the total weight content of the compounds of this group in the composition is less than or equal to 500 ppm, preferably less than or equal to 200 ppm, more particularly less than or equal to 100 ppm and ideally less than or equal to 50 ppm.

12. Process for producing 1,3,3,3-tetrafluoropropene, especially in trans form, comprising:
- the provision of a composition according to one of Claims 1 to 11;
- the reaction of this composition with hydrofluoric acid, preferably in the gas phase.

13. Process according to Claim 12, comprising a single step of catalytic fluorination.

14. Process according to Claim 12, comprising two successive steps of catalytic fluorination, namely:
- the reaction of the composition with hydrofluoric acid in the gas phase, to manufacture an intermediate product;
- optionally, purification of the intermediate product; and then
- the reaction of the intermediate product with hydrofluoric acid in the gas phase, to manufacture 1,3,3,3-tetrafluoropropene;
the intermediate product preferably being 1-chloro-3,3,3-trifluoropropene, especially in trans form.
